# EUROPÄISCHE PATENTSCHRIFT

(11) **EP 1 128 771 B1**
(45) Veröffentlichungstag und Bekanntmachung des Hinweises auf die Patenterteilung: **30.08.2006**
(21) Anmeldenummer: 00943628.8
(22) Anmeldetag: 20.05.2000
(51) Int. Cl.: A61B 17/70

(54) **KNOCHENSCHRAUBE ZUR WINKELVARIABLEN VERBINDUNG MIT EINEM LÄNGSTRÄGER**
ORTHOPAEDIC SCREW FOR VARIABLE ANGLE CONNECTION TO A LONGITUDINAL SUPPORT
VIS A OS A ASSEMBLER A UN ANGLE VARIABLE AVEC UN SUPPORT LONGITUDINAL

(30) Priorität: 15.09.1999 DE 19944120
(43) Veröffentlichungstag der Anmeldung: 05.09.2001
(73) Patentinhaber: Ulrich GmbH & Co. KG, 89081 Ulm (DE)
(72) Erfinder: KLUGER, Patrick, D-89155 Erbach (DE)
(74) Vertreter: Hentrich, Swen
(86) Internationale Anmeldenummer: PCT/DE2000/001658
(87) Internationale Veröffentlichungsnummer: WO 2001/019266

(56) Entgegenhaltungen:
- WO-A-94/00066
- DE-A- 19 534 136
- DE-U- 29 712 697

## Beschreibung

Die Erfindung betrifft eine Knochenschraube zur winkelvariablen Verbindung mit einem Längsträger für die Osteosynthese, insbesondere Pedikelschraube für Implantate zur Korrektur und Stabilisierung der Wirbelsäule, mit einem Gewindefuß zum Einschrauben in den Knochen, insbesondere Wirbelkörper, und mit einem zur Befestigung des Längsträgers gestalteten Schraubenkopf.

Derartige Knochenschrauben sind aus der Praxis bekannt, wobei diese zur Fixierung eines aus einer Mehrzahl möglicher Winkelstellungen ausgewählten Winkels eine symmetrisch um die Drehachse ausgebildete Verzahnung aufweisen, die jedoch nur aufwendig und damit kostenintensiv zu fertigen ist und darüberhinaus auch lediglich eine endliche Anzahl von in diskreten Abständen angeordneten Winkelstellungen ermöglicht.

DE-U-29712697 offenbart eine Knochenschraube mit den Merkmalen des Oberbegriffs von Anspruch 1.

Pedikelschrauben sind beispielsweise aus der DE 41 07 480 C2 bekannt, wobei der Gewindefuß und der Schraubenkopf zweiteilig und über eine Schwalbenschwanzführung miteinander verbindbar ausgeführt sind. Der Schraubenkopf ist dabei durch ein Bügelteil gebildet, in dem nach dessen Aufschieben auf den Schwalbenschwanz durch eine Madenschraube der Längsträger fixiert wird. Derartige Pedikelschrauben haben sich in der Praxis gut bewährt; allerdings hat es sich in bestimmten Anwendungsfällen als wünschenswert gezeigt, wenn nach dem Einschrauben der Pedikelschraube in den Wirbelkörper mit wenig Einzelteilen und Handgriffen eine Anpassung und nachfolgende Sicherung der Lage des Längsträgers erfolgen kann.

Der Erfindung liegt daher die Aufgabe zugrunde, eine Knochenschraube der eingangs genannten Art so auszubilden, daß auch nach deren Plazierung die Lage des Längsträgers relativ zu deren Längsachse frei geändert werden kann.

Diese Aufgabe wird bei einer Knochenschraube der eingangs genannten Art dadurch gelöst, daß der Schraubenkopf als Teilkugel mit einem eine Gewindebohrung aufweisenden Kegelstumpf gestaltet ist, daß der Kegelstumpf mit seiner Gewindebohrung geneigt zur Längsachse des Gewindefußes ausgerichtet ist, und daß auf dem Kegelstumpf mittels einer in die Gewindebohrung eingreifenden Schraube eine eine Aufnahme für den Längsträger bereitstellende Klemmschelle befestigbar ist.

Die Erfindung bietet den Vorteil, daß die Klemmschelle mit gelöster Schraube um den Kegelstumpf verdrehbar und der Längsträger in der Aufnahme der Klemmschelle in seiner Längsrichtung verschiebbar ist, also der Operateur sowohl die Orientierug als auch die relative Erstreckung des Längsträgers gegenüber dem Knochen einfach verändern kann. Sobald die gewünschte Position mit frei wählbarer Winkelstellung erreicht ist, wird durch Anziehen der Schraube zugleich die Bewegungsfreiheit hinsichtlich der Drehung und Verschiebung beendet. Einer Verzahnung bedarf es dabei nicht, weil der Kegelstumpf eine gegenüber der Drehachse der in die Gewindebohrung eingreifenden Schraube geneigte Mantelfläche besitzt, so daß die Belastungsmomente der Verbindung von Klemmschelle und Knochenschraube nicht mehr nur durch Reibung oder den Formschluß der Verzahnung aufgefangen werden müssen, sondern über den Kegelstumpf auf eine Fläche verteilt und auf eine Biegebelastung in Klemmschelle bzw Längsträger übertragen werden.

Zweckmäßigerweise ist dabei vorgesehen, daß der Kegelstumpf von einem an der Teilkugel ausgebildeten Anlagerand für die Klemmschelle umgeben ist, da so eine größere, nicht auf die Flanke des Kegelstumpfes beschränkte Anlagefläche zur Verfügung steht.

Eine bevorzugte Ausführungsform der Erfindung ist dadurch gekennzeichnet, daß die Klemmschelle durch eine Biegeklammer gebildet ist, deren parallel zueinander verlaufende Schenkel durch einen die Aufnahme bereitstellenden Federring verbunden sind und Öffnungen für den Durchgriff der Schraube aufweisen. Diese Ausführungsform bietet den Vorteil, daß zu unterschiedlichen Stadien des Festziehens der Schraube die Drehbeweglichkeit der Klemmschelle oder die Axialverschieblichkeit des Längsträgers beschränkt oder beendet werden kann.

Es hat sich als günstig gezeigt, wenn die Wandung der Öffnungen eine zu der Neigung des Kegelstumpfes korrespondierende Neigung besitzen, da es so nicht lediglich zu einer Linienberührung mit entsprechend hohen Flächenpressungen kommt, sondern vielmehr eine größere Anlagefläche zur Verfügung steht.

Wenn die Außenwand des Kegelstumpfes eine in Umfangsrichtung verlaufende Kehlung aufweist, besteht die Möglichkeit, daß die Neigung der Klemmschelle zumindest begrenzt vor dem Festziehen der Schraube verändert werden kann.

Hinsichtlich einer maximierten Fläche des Anlagerandes ist es günstig, wenn die Teilkugel durch eine in den Gewindefuß übergehende Halbkugel gebildet ist.

Im folgenden wird die Erfindung an in der Zeichnung dargestellten Ausführungsbeispielen näher erläutert; es zeigen:

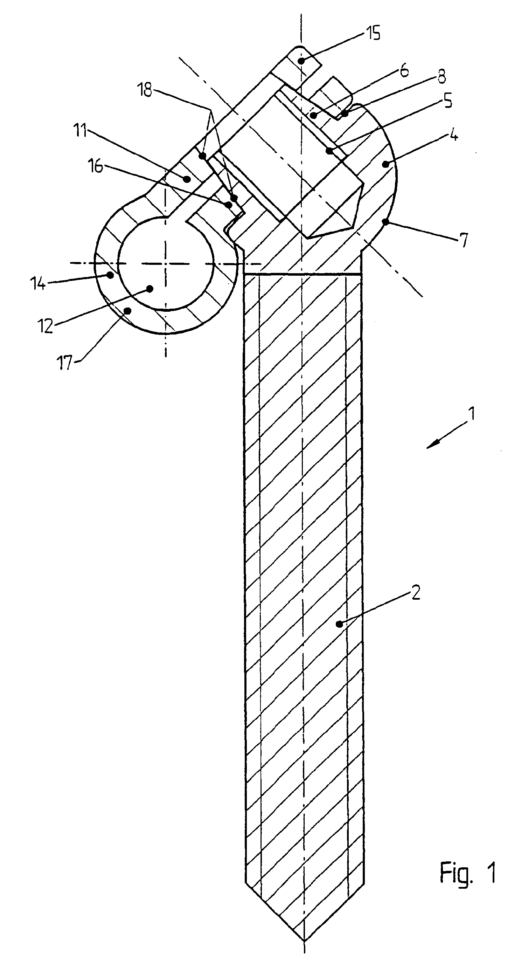

- Fig. 1: einen Axialschnitt durch eine erfindungsgemäße, als Pedikelschraube ausgeführte Knochenschrauben mit der auf dem Kegelstumpf angeordneten Klemmschelle,

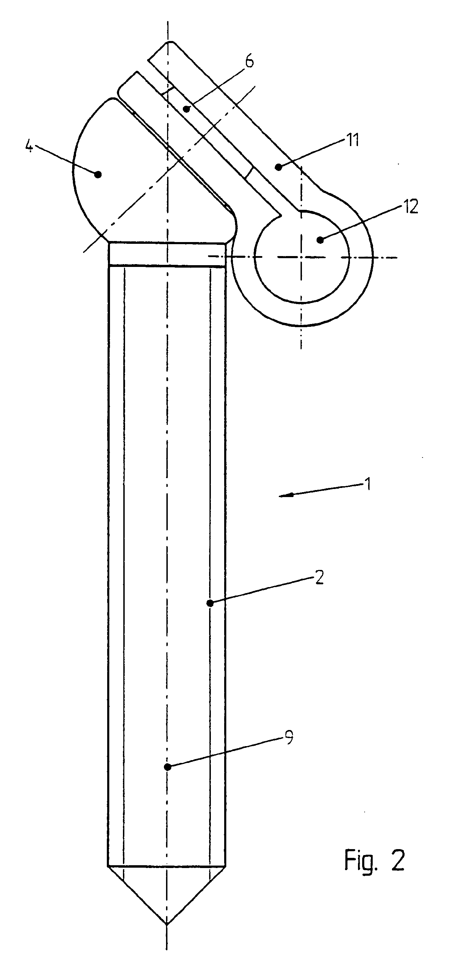

- Fig. 2: eine Seitenansicht der Pedikelschraube mit Klemmschelle,

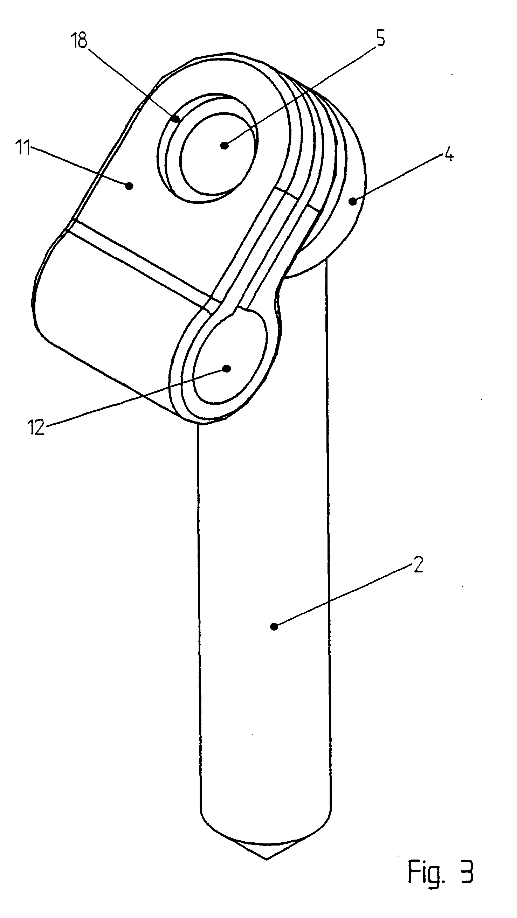

- Fig. 3: eine perspektivische Darstellung der Pedikelschraube mit Klemmschelle,

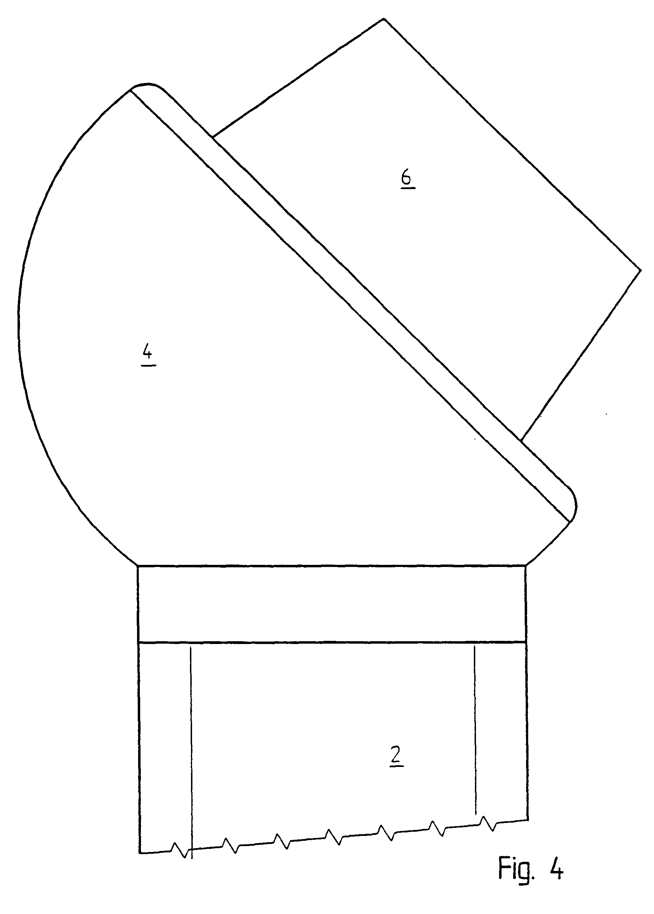

- Fig. 4: eine Seitenansicht des Schraubenkopfes mit dem Kegelstumpf und dem Anlagerand,
- Fig. 5: eine der Fig. 4 entsprechende Darstellung, mit einer in Umfangsrichtung des Kegelstumpfes umlaufenden Kehlung,
- Fig. 6: eine Vorderansicht zweier in einen Wirbel eingeschraubter, je einen Längsträger haltender Pedikelschrauben,
- Fig. 7: eine Ansicht aus Richtung des Pfeiles VII aus Fig. 6, und

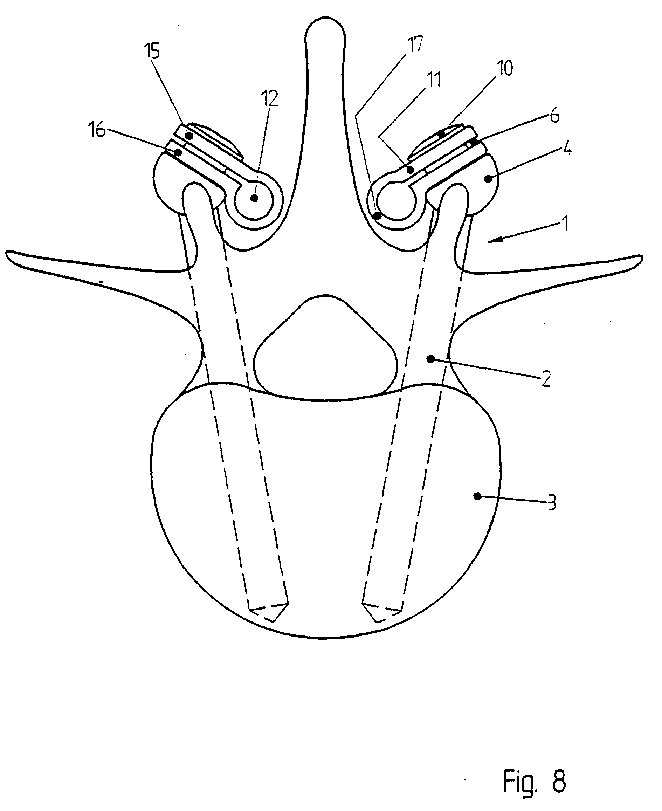

- Fig. 8: eine Ansicht aus Richtung des Pfeiles VIII aus Fig. 6.

Die erfindungsgemäße Knochenschraube ist für den Einsatz in der Osteosynthese vorgesehen, wenn eine winkelvariable Verbindung mit einem Längsträger gewünscht ist, der beispielsweise in der Wirbelsäulenchirurgie Verwendung findet, wobei dann die Knochenschraube als Pedikelschraube 1 bezeichnet ist. Die in der Zeichnung dargestellte Pedikelschraube 1 dient zur Befestigung von Implantaten, mit denen die Wirbelsäule korrigiert und stabilisiert werden soll. Die Pedikelschraube 1 weist einen Gewindefuß 2 zum Einschrauben in den Wirbelkörper 3 und einen Schraubenkopf 4 auf, der als Teilkugel mit einem eine Gewindebohrung 5 aufweisenden Kegelstumpf 6 ausgebildet ist, wobei die Teilkugel bei der in der Zeichnung dargestellten Ausführungsform als eine in den Gewindefuß 2 übergehende Halbkugel 7 realisiert ist, deren Durchmesser so bestimmt ist, daß der Kegelstumpf 6 von einen Anlagerand 8 umgeben ist. Der Kegelstumpf 6 ist mit seiner Gewindebohrung 5 geneigt zur Längsachse 9 des Gewindefußes 2 ausgerichtet. Auf dem Kegelstumpf 6 und dem Anlagerand 8 ist mittels einer in die Gewindebohrung 5 eingreifenden Schraube 10 eine Klemmschelle 11 befestigt, die eine Aufnahme 12 für einen Längsträger 13 bereitstellt.

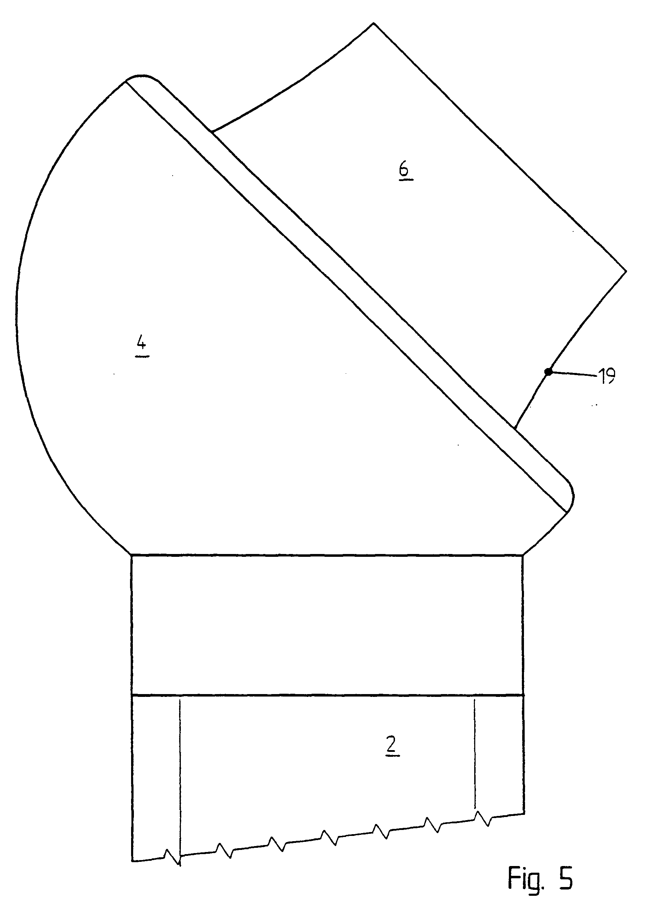

Die Klemmschelle 11 ist durch eine Biegeklammer 14 gebildet, deren parallel zueinander verlaufende Schenkel 15,16 durch einen die Aufnahme 12 bereitstellenden Federring 17 verbunden sind und Öffnungen 18 für den Durchgriff der Schraube 10 aufweisen. Die Wandung der Öffnungen 18 besitzt eine zu der Neigung des Kegelstumpfes 6 korrespondierende Neigung, wobei bei der Ausführungsform aus Fig. 5 weiterhin eine in Umfangsrichtung verlaufende Kehlung 19 vorhanden ist.

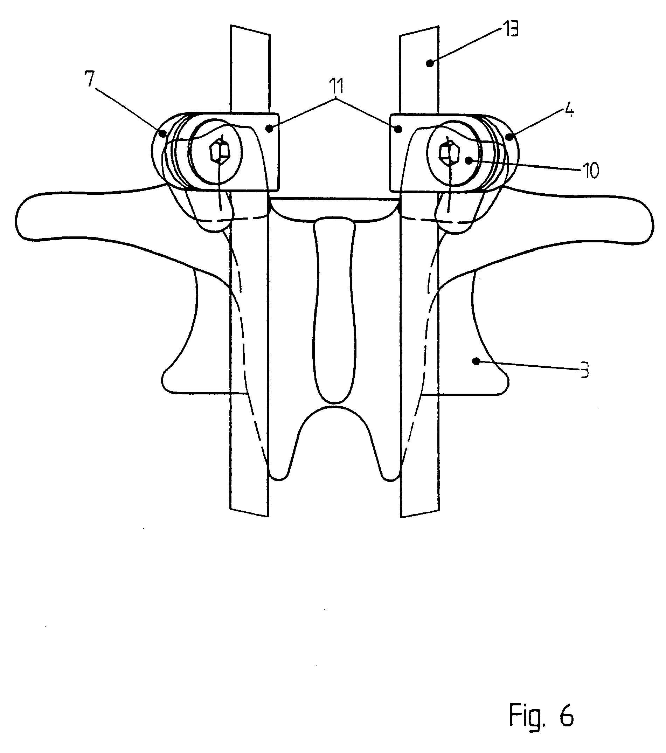

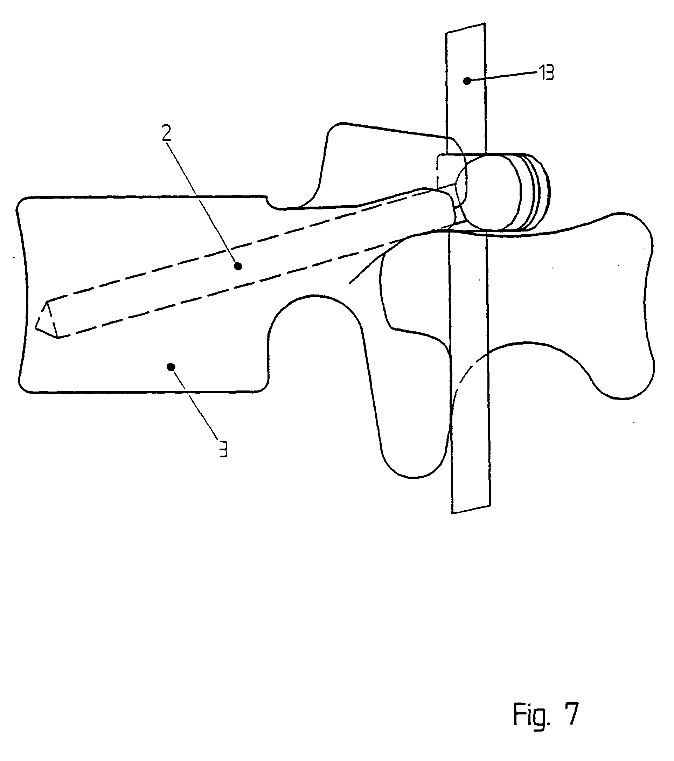

In den Fig. 6 und 7 ist gezeigt, wie die Pedikelschraube 1 genutzt wird, um mittels zweier an der Wirbelsäule entlanggeführter Längsträger 13 die Wirbelsäule zu stabilisieren und/oder zu korrigieren, wobei die Ausgestaltung der Pedikelschraube 1 eine vereinfachte Handhabung im Operationsfeld ermöglicht, da aufgrund der Neigung des Kegelstumpfes 6 die in seine Gewindebohrung 5 eingesetzte Schraube 10 leichter zugänglich ist und vom Operateur über größere Drehwinkel verstellt werden kann. Beim Festziehen dieser Schraube 10 wird simultan über einen Reibschluß die zuvor frei wählbare Drehlage der Klemmschelle 11 und die axiale Lage des Längsträgers 13 in der Aufnahme 12 fixiert.

## Patentansprüche

1. Knochenschraube zur winkelvariablen Verbindung mit einem Längsträger für die Osteosynthese, insbesondere Pedikelschraube für Implantate zur Korrektur und Stabilisierung der Wirbelsäule, mit einem Gewindefuß (2) zum Einschrauben in den Wirbelkörper (3) und mit einem zur Befestigung eines Längsträgers (13) als Teilkugel gestalteten Schraubenkopf (4), **dadurch gekennzeichnet, daß** der Schraubenkopf (4) mit einem eine Gewindebohrung (5) aufweisenden Kegelstumpf (6) gestaltet ist, daß der Kegelstumpf (6) mit seiner Gewindebohrung (5) geneigt zur Längsachse (9) des Gewindefußes (2) ausgerichtet ist, und daß auf dem Kegelstumpf (6) mittels einer in die Gewindebohrung (5) eingreifenden Schraube (10) eine eine Aufnahme (12) für den Längsträger (13) bereitstellende Klemmschelle (11) befestigbar ist.

2. Knochenschraube nach Anspruch 1, **dadurch gekennzeichnet, daß** der Kegelstumpf (6) von einem an der Teilkugel ausgebildeten Anlagerand (8) für die Klemmschelle (11) umgeben ist.

3. Knochenschraube nach Anspruch 1 oder 2, **dadurch gekennzeichnet, daß** die Klemmschelle (11) durch eine Biegeklammer (14) gebildet ist, deren parallel zueinander verlaufende Schenkel (15,16) durch einen die Aufnahme (12) bereitstellenden Federring (17) verbunden sind und Öffnungen (18) für den Durchgriff der Schraube (10) aufweisen.

4. Knochenschraube nach Anspruch 3, **dadurch gekennzeichnet, daß** die Wandung der Öffnungen (18) eine zu der Neigung des Kegelstumpfes (6) korrespondierende Neigung besitzen.

5. Knochenschraube nach einem der Ansprüche 1 bis 3, **dadurch gekennzeichnet, daß** die Außenwand des Kegelstumpfes (6) eine in Umfangsrichtung verlaufende Kehlung (19) aufweist.

6. Knochenschraube nach einem der Ansprüche 1 bis 5, **dadurch gekennzeichnet, daß** die Teilkugel durch eine in den Gewindefuß (2) übergehende Halbkugel (7) gebildet ist.

## Claims

1. A bone screw for variable-angle connection to a longitudinal support for osteosynthesis, in particular a pedicle screw for implants for correcting and stabilising the spinal column, comprising a screwthreaded shank (2) for screwing into the vertebra (3), and a screw head (4) in the form of a partial sphere for securing a longitudinal support (13), **characterised in that** the screw head (4) is shaped with a frustoconical portion (6) having a screwthreaded bore (5), the frustoconical portion (6) with its screwthreaded bore (5) is oriented inclinedly relative to the longitudinal axis (9) of the screwthreaded shank (2), and a clamping clip (11) forming a receiving means (12) for the longitudinal support (13) can be secured on the frustoconical portion (6) by means of a screw (10) engaging into the screwthreaded bore (5).

2. A bone screw according to claim 1 **characterised in that** the frustoconical portion (6) is surrounded by a support edge (8) provided on the partial sphere for the clamping clip (11).

3. A bone screw according to claim 1 or claim 2 **characterised in that** the clamping clip (11) is formed by a bending clip (14) whose mutually parallel legs (15, 16) are connected by a spring ring (17) providing the receiving means (12) and have openings (18) for the screw (10) to engage therethrough.

4. A bone screw according to claim 3 **characterised in that** the wall of the openings (18) have an inclination which corresponds to the inclination of the frustoconical portion (6).

5. A bone screw according to one of claims 1 to 3 **characterised in that** the outer wall of the frustoconical portion (6) has a circumferentially extending recess (19).

6. A bone screw according to one of claims 1 to 5 **characterised in that** the partial sphere is formed by a hemisphere (7) merging into the screwthreaded shank (2).

## Revendications

1. Vis à os pour assembler selon un angle variable un support longitudinal pour une ostéosynthèse, notamment une vis à pédicule destinée à des implants de correction et de stabilisation de la colonne vertébrale, cette vis comportant un pied fileté (2) pour vissage dans la vertèbre (3) et une tête de vis (4) avec une partie conique pour fixer un support longitudinal (13), **caractérisée en ce que** la tête de vis (4) présente un tronc de cône (6) percé d'un alésage fileté (5), que ce tronc avec son alésage est incliné par rapport à l'axe longitudinal (9) du pied fileté (2), et qu'une bride de pincement (11) présentant un logement (12) pour le support longitudinal (13) peut être fixé sur le tronc de cône (6) par une vis (10) engagée dans l'alésage fileté (5).

2. Vis à os selon la revendication 1, **caractérisée en ce que** le tronc de cône (6) est entouré par un bord d'appui (8) réalisé sur la partie sphérique pour la bride de pincement (11).

3. Vis à os selon la revendication 1 ou 2, **caractérisée en ce que** la bride de pincement (11) a la forme d'une bride de flexion (14) dont les branches (15, 16) parallèles entre elles sont reliées par une bague élastique (17) présentant le logement (12) et sont percées d'ouvertures (18) pour le passage de la vis (10).

4. Vis à os selon la revendication 3, **caractérisée en ce que** la paroi des ouvertures (18) présente une inclinaison correspondant à celle du tronc de cône (6).

5. Vis à os selon une des revendications 1 à 3, **caractérisée en ce que** la paroi externe du tronc de cône (6) présente une dépression (19) s'étendant périphériquement.

6. Vis à os selon une des revendications 1 à 5, **caractérisée en ce que** la partie sphérique est constituée par une demi-sphère (7) se poursuivant dans le pied fileté (2).
